# EUROPEAN PATENT SPECIFICATION

(11) **EP 1 940 396 B1**
(45) Date of publication and mention of the grant of the patent: **06.07.2011**
(21) Application number: 05790713.1
(22) Date of filing: 15.09.2005
(51) Int. Cl.: A61K 31/4184, A01N 43/52, A61P 33/10, A01N 43/04, A61K 9/08

(54) **ANTHELMINTIC FORMULATIONS**
ANTHELMINTISCHE FORMULIERUNGEN
COMPOSITIONS AUX ANTHELMINTHIQUES

(43) Date of publication of application: 09.07.2008
(73) Proprietor: Merial Limited, Harlow, Essex CM19 5TG (GB)
(72) Inventor: RAZZAK, Majid Hameed Abdul, Auckland, 1309 (NZ)
(74) Representative: Harding, Charles Thomas
(86) International application number: PCT/NZ2005/000243
(87) International publication number: WO 2007/032688

(56) References cited:
- EP-A2- 0 136 033
- EP-A2- 0 137 627
- WO-A1-01/05232
- WO-A1-01/60380
- WO-A1-95/05812
- WO-A1-03/092680
- WO-A1-2004/043445
- WO-A2-00/61068
- FR-A- 2 755 824
- NZ-A- 329 247
- US-A1- 2005 245 468

## Description

### FIELD OF THE INVENTION

This invention relates to liquid solutions of triclabendazole and in particular to veterinary anthelmintic formulations including triclabendazole in liquid solution, it has particular, though no sole, application to liquid pour-on formulations.

### BACKGROUND OF THE INVENTION

Diseases caused by parasitic helminths such as nematodes, cestodes and trematodes (liver fluke) can cause severe economic losses in the farming of ruminants and other animals. Fasciolisis, a disease caused by parasitic liver flukes, is commonly contracted by domestic herbivorous animals such as sheep, cattle and goats. The liver flukes Fasciola hepatica and Fasciola gigantica are flat worms which live in the bile ducts (in the liver) of their hosts.

Fasciola hepatica infection is widespread and is generally associated with low lying wet or water-covered areas. Areas where the average annual rainfall is at or above about 600mm and irrigation areas in particular, tend to create ideal living/propagating environments for aquatic snails which serve as intermediate hosts for first larvae of fasciola hepatica (miracidia). The miracidia develop and multiply and eventually leave the snail host and encyst on vegetation forming metacercarial (infective stage of fasciola hepatica). When the vegetation is consumed by a grazing animal, the metacercarial excysts in the small intestine releasing the young parasite. These immature flukes penetrate the intestinal wall and enter the abdominal cavity. From there they migrate to the liver. Acute infections can result from the immature flukes burrowing through the liver substance. Death often follows as a result of concomitant blood loss.

It has been estimated that around the world at least 40 million sheep and 6 million cattle graze on pastures contaminated with fasciola hepatica (liver fluke).

Because ruminants, such as sheep, cattle and goats, are susceptible to fasciolisis, it is of critical importance that formulations effective against the disease are available.

Until recently, the treatment of fasciolisis has been less than optimal. The drug previously used, bithionol, had to be administered orally over five days at a dosage of 30 mg/kg of body weight Praziquantel, a very effective drug against most trematode infections, is inactive against Fasciola species.

The benzimidazole class of active agents are known for their anthelmintic activity. They are known to be sparingly soluble and are either made up in tablet or powder form (for use with small animals) or typically made up as suspensions for use in oral drenches.

Of all the benzimidazoles known, triclabendazole, which is a halogenated benzimidazole, is highly effective against liver flukes (fasciolisis) at all stages of their life cycle. Triclabendazole is known as 5-Chloro-6-(2,3-dichlorophenoxy)-2-methylthio-1H-benzimidazole, and is represented by the following structural formula:

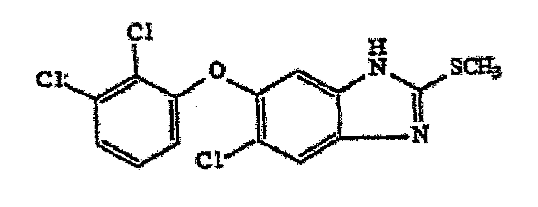

Other active agents within the benzimidazole class of actives such as albendazole are only effective against adult fluke.

In general for ease of application to large numbers of animals, farmers and veterinary surgeons prefer to treat farm animals with pour-ons rather than oral drenches or injectables. Pour-ons are typically relatively viscous liquids applied in small doses to the neck or back line of the animal. In the case of sheep pour on, the applicator guns are adapted to supply a dose of about 5ml to 10ml, and in the case of cattle the dose (depending upon the product) is typically about 40ml to 60ml. Most pour-ons have the active in solution, and the solvent or solvent system is chosen to allow the active to pass through the dermal layer and provide a sufficiently high, systemic amount of the active quickly enough.

The solvent system needs to be non-irritant to the farmer and the animal, stable, non-toxic, non-carcinogenic, and capable of being used in a pour-on applicator gun. Because of the small volume of each dose (too much would result in liquid running off the animal's back) the pour-on formulation is typically designed so that sufficient active is contained within the solvent system that a typical designed dose rate of 1ml of pour-on for each 10kg of animal live weight is standard. Thus a 10ml dose of a pour-on would normally supply sufficient active to treat a 100kg sheep, 50ml would supply normally enough active to treat a 500kg cattle beast.

Parasitologists on the other hand recommend the use of oral drenches as there is usually a better take up of active from an oral drench than a pour-on.

Formulators prefer to design solutions rather than suspensions for veterinary purposes, as solutions can normally be used for either the pour-on route of administration or the oral route of administration, and solutions are usually far more stable than suspensions when stored in bulk. However, if the active is known to be sparingly soluble in water or most practical solvents then the formulator will favour a suspension for use as an oral drench.

It is particularly advantageous to provide liquid formulations which contain a sufficient quantity of an active anthelmintic agent in a solution which can be easily administered by way of a pour-on.

It has been difficult to provide liquid formulations containing triclabendazole in solution. Indeed, until recently it has only been available as a suspension formulation for oral administration.

Commercial drench formulations of triclabendazole are known in which triclabendazole is suspended in a liquid carrier. Such formulations are administered orally via an appropriate drenching apparatus or by subcutaneous injection. Oral triclabendazole suspension formulations are commercially available and marketed under the trade name 'Fasinex'120 or 240. Such formulations can have a triclabendazole concentration in the order of 120g/l (or 240g/l), which equates to 12% w/v (24% w/v). A 50 ml (25ml) dose of Fasinex 120 (240) equates to a dose of 6g triclabendazole/500kg beast (12mg/kg).

While oral (drench) suspension formulations containing triclabendazole have been available and successful in treating fasciolisis, the method of administering a suitable volume of oral formulation often requires a suitably experienced/qualified person to administer a dose. Dosing a herd of animals can therefore be laborious, time consuming and place a severe economic strain on a conventional farm. Treatment of fasciolisis by way of a single injection represents an even less practical option for a farmer.

Pour-on formulations containing a benzimidazole compound are therefore desirable within the farming community but attempts to produce pour ons containing benzimidazoles have been unsuccessful until the applicant invented a formulation for a triclabendazole pour on. The applicant had previously tried unsuccessfully to formulate an oxfendazole pour on.

In WO95/23590 (Bomac), a pour-on formulation is described in which a benzimidazole, selected from oxfendazole and/or albendazole, is formulated in a suspension. Suspensions of albendazole in accordance with formulations described in 'Bomac' have not been effective in the treatment of a parasitic burden. In any case as stated earlier albendazole is not the preferred active for treatment of liver fluke.

Other pour-on formulations have been suggested in patent literature in which an active agent is dissolved; emulsified; or suspended in a solvent/solvent mixture. Anthelmintic formulations are known which contain oxfendazole; tetramisole and levamisole that exhibit anthelmintic action as a pour-on comparable to that of analogous oral or injectable treatments. French patent registration no. 96 14068 (publication number FR2755824A) teaches a formulation for topical administration including oxfendazole in an amount of 5% w/v dissolved in a non-aqueous 'vehicle', a non-aqueous cosolvent, a non-ionic surfactant and a polymer. As stated earlier, however, such anthelmintics are not effective in the treatment of early immature and immature fasciola hepatica.

WO 03/92680 describes surfactant-based injection, oral liquid formulation or oral cream, which contains triclabendazole which may be used in combination with macrolide anthelmintics.

WO 2004043445 describes aqueous micellar formulations of benzimidazoles in combination with macrocyclic lactones for topical administration.

WO 01/60380 describes a parasiticidal formulation including a mixture of a pyrrolidine solvent, a bridging solvent and at least one parasiticidal agent.

The solubility characteristic of triclabendazole makes formulating an effective triclabendazole pour-on extremely difficult. A number of rate limiting barriers are faced in formulating active ingredient(s) in a commercially effective dosage form including (i) efficient and sufficient absorption to provide systemic amounts of active quickly; (ii) minimal exposure of an animal to a toxic dosage form; and (iii) stability of formulation.

These and other limitations are amplified given the characteristics of triclabendazole and the desire to provide a triclabendazole pour-on formulation, which is additionally required to permeate across an animal's dermis, and be sufficiently absorbed and transported to the site of infection (liver). Even further barriers exist to the extent that a sufficient kill rate of liver fluke needs to be achieved to substantially minimise potential development of drug resistant parasitic strains.

For triclabendazole pour-on an effective dose is a dose of about 30 mg per kg animal weight. If the triclabendazole is present in solution at 150g per ml or 15%w/v, a dose of 70 ml will deliver sufficient triclabendazole to treat fasciolisis in a cow weighing 350 kg.

In PCT/NZ00/00053 we disclose certain solvents which are capable of dissolving triclabendazole. The specific solvents disclosed are benzyl alcohol, n-methyl pyrrolidone, glycol ethers including butyl dioxitol. The formulations included as examples incorporated triclabendazole at 30 % w/v. Thus a dose of 35 ml will deliver sufficient triclabendazole to treat fascioliasis in a cow weighing 350 kg.

While this is a significant advance it would be useful to be able to include triclabendazole in solution with other solvents for pour-ons and for-other routes of administration, and in the case of a pour on formulation preferably containing higher concentrations of triclabendazole to allow an even smaller volume of formulation to be applied to the animal while still providing an effective dose.

While reference has been made to prior art in the specification it is not to be taken as an admission that the cited art forms part of the common general knowledge.

### OBJECT OF THE INVENTION

It is an object of the invention to provide an improved veterinary anthelmintic formulation including triclabendazole in solution, or one which will at least provide the public with a useful choice.

### SUMMARY OF THE INVENTION

In one aspect the invention provides a veterinary anthelmintic formulation containing a stable solution of triclabendazole, wherein the solution contains an effective amount of triclabendazole and a solvent system containing one or more solvents, wherein at least one of the solvents is selected from the group comprising 2-pyrrolidone and liquid polyethylene glycol, and wherein the triclabendazole is present in the range 40-60% w/v.

Preferably the solvent system also includes one or more additional solvents selected from the group comprising benzyl alcohol, propylene glycol, N-methyl pyrrolidone, glycerol formal, glycol ethers, butyl dioxitol, methyl alcohol, ethyl alcohol, isopropyl alcohol, 1-butanol and 1-hexanol.

In its most preferred aspect the invention provides a -veterinary pour-on anthelmintic formulation containing a stable solution of triclabendazole, wherein the solution contains an effective amount of triclabendazole and a solvent system containing one or more solvents, wherein at least one of the solvents is selected from the group comprising 2-pyrrolidone and liquid polyethylene glycol, wherein the triclabendazole is present in the range 40-60% w/v.

Preferably the solvent system also includes one or more additional solvents selected from the group comprising benzyl alcohol, propylene glycol, N-methyl pyrrolidone; glycerol formal, glycol ethers, butyl dioxitol, methyl alcohol, ethyl alcohol, isopropyl alcohol, 1-butanol and 1-hexanol.

Preferably the solvent system contains 2-pyrrolidone and at least one additional solvent selected from the group comprising butyl dioxitol, N-methyl pyrrolidone, benzyl alcohol, glycerol formal, propylene glycol and ethyl alcohol.

Preferably the triclabendazole is present in a concentration such that an effective amount of triclabendazole may be delivered to an animal in a volume of substantially 25 ml or less.

Preferably the formulation includes at least one additional anthelmintic active or other medicament which is soluble in the solvent system.

Preferably the additional anthelmintic active or actives are selected from the group comprising avermectins, milbemycins, tetramisole and levamisole.

Preferably the additional anthelmintic active is an avermectin, such as abamectin.

Preferably the formulation further includes excipients such as dyes, preservatives, stabilisers, buffers, thickeners, anti-foaming agents, spreading agents and the like.

Preferably the formulation contains (a) about 60% w/v of triclabendazole, (b) about 1.0% w/v of abamectin, and a solvent system, wherein the solvent system includes N-methyl pyrrolidone in an amount of about 10% w/v and 2-pyrrolidone in an amount of about 29% w/v.

In another aspect the invention provides a veterinary anthelmintic formulation for use in treating parasites including fasciolisis in warm blooded animals.

In another aspect the invention provides a veterinary anthelmintic formulation for use in treating parasites including fasciolisis in warm blooded animals in the form of a pour on formulation as described above for administration in a volumne of about 25 ml or less of the pour-on formulation on to the skin surface of an animal.

Preferably, the veterinary anthelmintic formulation for use in treating fasciolisis in warm blooded animals in the form of a pour on formulation is capable of delivering at least 1mg of triclabendazole per 10kg of body weight of the animal to be treated. References to 'medicaments' include those substances such as anthelmintics, antigens, vaccines, vitamin and mineral supplements and other substances, which may be useful for promoting the health of the animal.

'Liquid polyethylene glycol' includes polyethylene glycol (PEG) that is liquid at room temperature. PEG 200 to 400 are liquid at room temperature. In addition, blends containing polyethylene glycols of higher or lower molecular weight are useable if the resulting mixture is liquid at room temperature.

### PREFERRED EMBODIMENTS

Benzimidazoles are generally regarded as being difficult to dissolve, and consequently commercial formulations are made up as liquid suspensions mainly for application as oral drenches.

As a comparison we conducted trials to determine the solubility of various members of the benzimidazole family in different solvents. We found that oxfendazole, albendazole and ricobendazole are all either insoluble or so insignificantly soluble that they cannot be formulated as effective veterinary solutions in solvents such as benzyl alcohol, propylene glycol, NMP, 2-pyrollidone, PEG, glycerol formal and butyl dioxitol.

When we conducted trials with triclabendazole it was found that the triclabendazole was more soluble generally in the solvents mentioned above than the other benzimidazoles which were sparingly soluble at best, and in most cases insoluble. But more importantly we discovered that triclabendazole is extremely soluble in 2-pyrrolidone and PEG 400 with more than 500 mg of triclabendazole capable of being dissolved in a ml of these solvents. This is particularly advantageous as it allows for formulations of low volume to be formulated for delivery to cattle and other animals.

**Table 1a: Solubility Studies of triclabendazole in different organic solvents**

| Sample No. | Solvent Name | Quantity dissolved in 10 ml solvent | Total Volume occupied | Amount dissolved (mg/ml) |
|---|---|---|---|---|
| 1 | Benzyl Alcohol | 5.0 g (Max solubility) | 14 ml | 357.14 |
| 2 | Propylene Glycol | 1.5 g (Max solubility) | 11.0 ml | 136.36 |
| 3 | NMP^{α} | 4.5 g (Max solubility) | 14.0 ml | 321.43 |
| 4 | 2-pyrrolidone | 10.0 g (flowable in nature) | 17.5-18.0 ml | 555.55 |
| 5 | PEG400 | 10.0 g (flowable in nature) | 17.5 ml | 571.43 |
| 6 | Glyceryl Formal | 1g (Max solubility) | | 90.91 |
| 7 | DGBE (butyl dioxitol) | 2.0 g (Max solubility) | | 166.66 |

| | | | | |
|---|---|---|---|---|
| ^{α}NMP-N-methyl pyrrolidone | | | | |

**Table 1b: Solubility studies of triclabendazole in different alcohols**

| Sample No. | Solvent Name | Triclabendazol e taken in 10 ml solvent. Day 0 | Triclabendazole added to previous system. Day 1 | Triclabendazo le added to previous system. Day 7 | Solvent added to the previous system. Day 12 | Solvent added to the previous system. Day 15 |
|---|---|---|---|---|---|---|
| 1 | Methyl alcohol | 500 mg | 500 mg | 1 g | 5 ml | 5 ml |
| 2 | Ethyl alcohol | 500 mg | 500 mg | 1 g | 5 ml | 5 ml |
| 3 | Isopropyl alcohol | 500 mg | 500 mg | 1 g | 5 ml | --- |
| 4 | 1-butanol | 500 mg | 500 mg | 1 g | 5ml | --- |
| 5 | 1-hexanol | 500 mg | 500 mg | 1 g | 5 ml | --- |

**Table 1c: Solubility Studies of Triclabendazole is various alcohols**

| Sample No. | Solvent Name | Quantity dissolved in solvent) | Total volume occupied | Amount dissolved (mg/ml) |
|---|---|---|---|---|
| 1 | Methyl alcohol | 2 gm/20 ml | 22.5 ml | 88.88 |
| 2 | Ethyl alcohol | 2 gm/20 ml | 22.5 ml | 88.88 |
| 3 | Isopropyl alcohol | 2 gm/15 ml | 17.0 ml | 117.5 |
| 4 | 1-butanol | 2 gm/15 ml | 17.0 ml | 117.5 |
| 5 | 1-hexanol | 2 gm/15 ml | 17.0 ml | 117.5 |

Based on the above solubility data various high concentration formulations containing 2-pyrrolidone or PEG were proposed. The formulations were prepared and stored at room temperature and refrigeration temperature for 30 days. The formulations showed no deposition at room temperature or under refrigeration.

In field use these formulations would be highly desirable as they could provide the flexibility to deliver high concentrations of triclabendazole to the animal in a relatively small amount of solvent The ideal would be to deliver concentrated quantities of triclabendazole in a solvent delivered at the rate of as low as 1ml per 20kg bodyweight.

After conducting a number of solubility studies of benzimidazoles in general and triclabendazole in particular it has now been found that triclabendazole is extremely soluble in 2-pyrrolidone and liquid polyethylene glycol (particularly PEG 400), with more than 500 mg of triclabendazole dissolving in a millilitre of each of these solvents.

It has been found that stable liquid formulations containing triclabendazole in solution with these solvents can be prepared and that these actives can be absorbed through the skin to control parasitic diseases, including fasciolisis in warm blooded animals. The use of these particular solvents is advantageous as it allows high concentrations of triclabendazole to be dissolved so that doses of low volume can be administered to cattle and other animals.

Based on the solubility studies various formulations containing 2-pyrrolidone or liquid polyethylene glycol were prepared in accordance with the following examples.

The example formulations below are particularly suitable for administration as pour-ons. However, the solutions of the present invention are useable in different dosage forms. By way of example the triclabendazole solution may be incorporated into oral dosage forms as tablets, capsules (each of which includes sustained release or timed release formulations) and drenches. Other topical administration forms such as spot-ons are useable. The various dosage forms are well known to those of ordinary skill in the pharmaceutical and veterinary arts.

### Reference Example 1

| **Ingredients** | **Quantity** |
|---|---|
| Triclabendazole | 30 g |
| 2-pyrrolidone | 50 g |
| DGBE (butyl dioxitol) q. s. to | 100 ml |

To prepare the formulation add the triclabendazole and 2 pyrrolidone. Warm to 60°C and stir to dissolve. Cool mix to less than 25°C and dilute to volume with DGBE.

### Reference Example 2

| **Ingredients** | **Quantity** |
|---|---|
| Triclabendazole | 30 g |
| PEG 400 | 50 g |
| DGBE (butyl dioxitol) q. s. to | 100 ml |

To prepare the formulation add the triclabendazole and PEG 400 (polyethylene glycol 400). Warm to 60°C and stir to dissolve. Cool mix to less than 25°C and dilute to volume with DGBE.

### Example 3

| **Ingredients** | **Quantity** |
|---|---|
| Triclabendazole | 60g |
| 2-pyrrolidone q. s. to | 100 ml |

To prepare the formulation add the triclabendazole, and 2-pyrolidone. Warm to 70°C and stir to dissolve. Cool mix to less than 25°C and dilute to volume with 2-pyrrolidone.

### Example 4

| **Ingredients** | **Quantity** |
|---|---|
| Triclabendazole | 60g |
| N-methyl pyrrolidone | 10g |
| 2-pyrolidone q. s. to | 100 ml |

To prepare the formulation add the triclabendazole, NMP and 2-pyrrolidone. Warm to 70°C and stir to dissolve. Cool mix to less than 25°C and dilute to volume with 2-pyrrolidone.

### Example 5

| **Ingredients** | **Quantity** |
|---|---|
| Triclabendazole | 60g |
| Benzyl Alcohol | 10g |
| 2-pyrrolidone q. s. to | 100 ml |

To prepare the formulation add the triclabendazole, Benzyl Alcohol and 2-pyrrolidone. Warm to 70°C and stir to dissolve. Cool mix to less than 25°C and dilute to volume with 2-pyrrolidone.

### Example 6

| **Ingredients** | **Quantity** |
|---|---|
| Triclabendazole | 60g |
| DGBE | 10g |
| 2-pyrolidone q. s. to | 100 ml |

To prepare the formulation add the triclabendazole, DGBE and 2-pyrrolidone. Warm to 70°C and stir to dissolve. Cool mix to less than 25°C and dilute to volume with 2-pyrrolidone.

### Example 7

| **Ingredients** | **Quantity** |
|---|---|
| Triclabendazole | 60g |
| Glycerol Formal | 10g |
| 2-pyrrolidone q. s. to | 100 ml |

To prepare the formulation add the triclabendazole, Glycerol Formal and 2-pyrrolidone. Warm to 70°C and stir to dissolve. Cool mix to less than 25°C and dilute to volume with 2-pyrrolidone.

### Example 8

| **Ingredients** | **Quantity** |
|---|---|
| Triclabendazole | 40 g |
| PEG 400 q. s. to | 100 ml |

To prepare the formulation add the triclabendazole and PEG 400. Warm to 70°C and stir to dissolve. Cool mix to less than 25°C and dilute to volume with PEG 400.

### Example 9

| **Ingredients** | **Quantity** |
|---|---|
| Triclabendazole | 40g |
| DGBE | 10g |
| PEG 400 q. s. to | 100 ml |

To prepare the formulation add the triclabendazole, DGBE and PEG 400. Warm to 70°C and stir to dissolve. Cool mix to less than 25°C and dilute to volume with PEG 400.

### Example 10

| **Ingredients** | **Quantity** |
|---|---|
| Triclabendazole | 40g |
| 2-pyrrolidone q. s. to | 100 ml |

To prepare the formulation add the triclabendazole, and 2-pyrrolidone. Warm to 70°C and stir to dissolve. Cool mix to less than 25°C and dilute to volume with 2-pyrrolidone.

### Example 11

| **S. No.** | **Ingredients** | **Quantity** |
|---|---|---|
| 1 | triclabendazole | 40g |
| 2 | NMP | 10g |
| 3 | 2-pyrrolidone q. s. to | 100 ml |

To prepare the formulation add the triclabendazole, NMP and 2-pyrrolidone. Warm to 70°C and stir to dissolve. Cool mix to less than 25°C and dilute to volume with 2-pyrrolidone.

### Example 12

| **S. No.** | **Ingredients** | **Quantity** |
|---|---|---|
| 1 | Triclabendazole | 40g |
| 2 | Benzyl alcohol | 10g |
| 3 | 2-pyrrolidone q. s. to | 100 ml |

To prepare the formulation add the triclabendazole, benzyl alcohol and 2-pyrrolidone. Warm to 70°C and stir to dissolve. Cool mix to less than 25°C and dilute to volume with 2-pyrrolidone.

### Example 13

| **Ingredients** | **Quantity** |
|---|---|
| Triclabendazole | 40g |
| Glycerol formal | 10g |
| 2-pyrrolidone q. s. to | 100 ml |

To prepare the formulation add the triclabendazole, glycerol formal and 2-pyrrolidone. Warm to 70°C and stir to dissolve. Cool mix to less than 25°C and dilute to volume with 2-pyrrolidone.

### Example 14

| **Ingredients** | **Quantity** |
|---|---|
| Triclabendazole | 40g |
| Propylene glycol | 10g |
| 2-pyrrolidone q. s. to | 100 ml |

To prepare the formulation add the triclabendazole, propylene glycol and 2-pyrrolidone. Warm to 70°C and stir to dissolve. Cool mix to less than 25°C and dilute to volume with 2-pyrrolidone.

### Reference Example 15

| **Ingredients** | **Quantity** |
|---|---|
| Triclabendazole | 20g |
| 2-pyrrolidone q. s. to | 100 ml |

To prepare the formulation add the triclabendazole, and 2-pyrrolidone. Warm to 70°C and stir to dissolve. Cool mix to less than 25°C and dilute to volume with 2-pyrrolidone.

### Reference Example 16

| **Ingredients** | **Quantity** |
|---|---|
| Triclabendazole | 20g |
| NMP | 10g |
| 2-pyrrolidone q. s. to | 100 ml |

To prepare the formulation add the triclabendazole, NMP and 2-pyrrolidone. Warm to 74°C and stir to dissolve. Cool mix to less than 25°C and dilute to volume with 2-pyrrolidone.

### Reference Example 17

| **Ingredients** | **Quantity** |
|---|---|
| Triclabendazole | 20g |
| Benzyl alcohol | 10g |
| 2-pyrrolidone q. s. to | 100 ml |

To prepare the formulation add the triclabendazole, benzyl alcohol and 2-pyrrolidone. Warm to 70°C and stir to dissolve. Cool mix to less than 25°C and dilute to volume with 2-pyrrolidone.

### Reference Example 18

| **Ingredients** | **Quantity** |
|---|---|
| Triclabendazole | 20g |
| Glycerol formal | 10g |
| 2-pyrrolidone q. s. to | 100 ml |

To prepare the formulation add the triclabendazole, glycerol formal and 2-pyrrolidone. Warm to 70°C and stir to dissolve. Cool mix to less than 25°C and dilute to volume with 2-pyrrolidone.

### Reference Example 19

| **Ingredients** | **Quantity** |
|---|---|
| Triclabendazole | 20g |
| Propylene glycol | 10g |
| 2-pyrrolidone q. s. to | 100 ml |

To prepare the formulation add the triclabendazole, propylene glycol and 2-pyrrolidone. Warm to 70°C and stir to dissolve. Cool mix to less than 25°C and dilute to volume with 2-pyrrolidone.

### Reference Example 20

| **Ingredients** | **Quantity** |
|---|---|
| Triclabendazole | 20g |
| Ethyl alcohol | 10g |
| 2-pyrrolidone q. s. to | 100 ml |

To prepare the formulation add the triclabendazole, ethyl alcohol and 2-pyrrolidone. Warm to 70°C and stir to dissolve. Cool mix to less than 25°C and dilute to volume with 2-pyrrolidone.

### Reference Example 21

| **Ingredients** | **Quantity** |
|---|---|
| Triclabendazole | 10g |
| 2-pyrrolidone q. s. to | 100 ml |

To prepare the formulation add the triclabendazole, and 2-pyrrolidone. Warm to 70°C and stir to dissolve. Cool mix to less than 25°C and dilute to volume with 2-pyrrolidone.

### Reference Example 22

| **Ingredients** | **Quantity** |
|---|---|
| Triclabendazole | 10g |
| NMP | 10g |
| 2-pyrrolidone q. s. to | 100 ml |

To prepare the formulation add the triclabendazole, NMP and 2-pyrrolidone. Warm to 70°C and stir to dissolve. Cool mix to less than 25°C and dilute to volume with 2-pyrrolidone.

### Reference Example 23

| **Ingredients** | **Quantity** |
|---|---|
| Triclabendazole | 10g |
| Benzylalcohol | 10g |
| 2-pyrrolidone q. s. to | 100 ml |

To prepare the formulation add the triclabendazole, benzyl alcohol and 2-pyrrolidone. Warm to 70°C and stir to dissolve. Cool mix to less than 25°C and dilute to volume with 2-pyrrolidone.

### Reference Example 24

| **Ingredients** | **Quantity** |
|---|---|
| Triclabendazole | 10g |
| Glycerol formal | 10g |
| 2-pyrrolidone q. s. to | 100 ml |

To prepare the formulation add the triclabendazole, glycerol formal and 2-pyrrolidone. Warm to 70°C and stir to dissolve. Cool mix to less than 25°C and dilute to volume with 2-pyrrolidone.

### Reference Example 25

| **Ingredients** | **Quantity** |
|---|---|
| Triclabendazole | 10g |
| Propylene glycol | 10g |
| 2-pyrrolidone q. s. to | 100 ml |

To prepare the formulation add the triclabendazole, propylene glycol and 2-pyrrolidone. Warm to 70°C and stir to dissolve. Cool mix to less than 25°C and dilute to volume with 2-pyrrolidone.

### Reference Example 26

| **Ingredients** | **Quantity** |
|---|---|
| Triclabendazole | 10g |
| Ethyl alcohol | 10g |
| 2-pyrrolidone q. s. to | 100 ml |

To prepare the formulation add the triclabendazole, ethyl alcohol and 2-pyrrolidone. Warm to 70°C and stir to dissolve. Cool mix to less than 25°C and dilute to volume with 2-pyrrolidone.

### Reference Example 27

| **Ingredients** | **Quantity** |
|---|---|
| Triclabendazole | 5g |
| 2-pyrrolidone q. s. to | 100 ml |

To prepare the formulation add the triclabendazole, and 2-pyrrolidone. Warm to 70°C and stir to dissolve. Cool mix to less than 25°C and dilute to volume with 2-pyrrolidone.

### Reference Example 28

| **Ingredients** | **Quantity** |
|---|---|
| Triclabendazole | 5g |
| NMP | 10g |
| 2-pyrrolidone q. s. to | 100 ml |

To prepare the formulation add the triclabendazole, NMP and 2-pyrrolidone. Warm to 70°C and stir to dissolve. Cool mix to less than 25°C and dilute to volume with 2-pyrrolidone.

### Reference Example 29

| **Ingredients** | **Quantity** |
|---|---|
| Triclabendazole | 5g |
| Benzyl alcohol | 10g |
| 2-pyrrolidone q. s. to | 100 ml |

To prepare the formulation add the triclabendazole, benzyl alcohol and 2-pyrrolidone. Warm to 70°C and stir to dissolve. Cool mix to less than 25°C and dilute to volume with 2-pyrrolidone.

### Reference Example 30

| **Ingredients** | **Quantity** |
|---|---|
| Triclabendazole | 5g |
| Glycerol formal | 10g |
| 2-pyrrolidone q. s. to | 100 ml |

To prepare the formulation add the triclabendazole, glycerol formal and 2-pyrrolidone. Warm to 70°C and stir to dissolve. Cool mix to less than 25°C and dilute to volume with 2-pyrrolidone.

### Reference Example 31

| **Ingredients** | **Quantity** |
|---|---|
| Triclabendazole | 5g |
| Propylene glycol | 10g |
| 2-pyrrolidone q. s. to | 100 ml |

To prepare the formulation add the triclabendazole, propylene glycol and 2-pyrrolidone. Warm to 70°C and stir to dissolve. Cool mix to less than 25°C and dilute to volume with 2-pyrrolidone.

### Reference Example 32

| **Ingredients** | **Quantity** |
|---|---|
| Triclabendazole | 5g |
| Ethyl alcohol | 10g |
| 2-pyrrolidone q. s. to | 100 ml |

To prepare the formulation add the triclabendazole, ethyl alcohol and 2-pyrrolidone. Warm to 70°C and stir to dissolve. Cool mix to less than 25°C and dilute to volume with 2-pyrrolidone.

### Example 33

| **Ingredients** | **Quantity** |
|---|---|
| Triclabendazole | 40 g |
| PEG 400 q. s. to | 100 ml |

To prepare the formulation add the triclabendazole and PEG 400. Warm to 70°C and stir to dissolve. Cool mix to less than 25°C and dilute to volume with PEG 400.

### Example 34

| **Ingredients** | **Quantity** |
|---|---|
| *Triclabendazole | 40g |
| Glycerol formal | 10g |
| PEG 400 q. s. to | 100 ml |

To prepare the formulation add the triclabendazole, glycerol formal and PEG 400. Warm to 70°C and stir to dissolve. Cool mix to less than 25°C and dilute to volume with PEG 400.

### Reference Example 35

| **Ingredients** | **Quantity** |
|---|---|
| Triclabendazole | 20 g |
| PEG 400 q. s. to | 100 ml |

To prepare the formulation add the triclabendazole and PEG 400. Warm to 70°C and stir to dissolve. Cool mix to less than 25°C and dilute to volume with PEG 400.

### Reference Example 36

| **Ingredients** | **Quantity** |
|---|---|
| Triclabendazole | 20g |
| Glycerol formal | 10g |
| PEG 400 q. s. to | 100 ml |

To prepare the formulation add the triclabendazole, glycerol formal and PEG 400. Warm to 70°C and stir to dissolve. Cool mix to less than 25°C and dilute to volume with PEG 400.

### Reference Example 37

| **Ingredients** | **Quantity** |
|---|---|
| Triclabendazole | 20g |
| Benzyl alcohol | 10g |
| PEG 400 q. s. to | 100 ml |

To prepare the formulation add the triclabendazole, benzyl alcohol and PEG 400. Warm to 70°C and stir to dissolve. Cool mix to less than 25°C and dilute to volume with PEG 400.

### Reference Example 38

| **Ingredients** | **Quantity** |
|---|---|
| Triclabendazole | 20g |
| Propylene glycol | 10g |
| PEG 400 q. s. to | 100 ml |

To prepare the formulation add the triclabendazole, propylene glycol and PEG 400. Warm to 70°C and stir to dissolve. Cool mix to less than 25°C and dilute to volume with PEG 400.

### Reference Example 39

| **Ingredients** | **Quantity** |
|---|---|
| Triclabendazole | 20g |
| Ethyl alcohol | 10g |
| PEG 400 q, s. to | 100 ml |

To prepare the formulation add the triclabendazole, ethyl alcohol and PEG 400. Warm to 70°C and stir to dissolve. Cool mix to less than 25°C and dilute to volume with PEG 400.

### Reference Example 40

| **Ingredients** | **Quantity** |
|---|---|
| Triclabendazole | 10 g |
| PEG 400 q. s. to | 100 ml |

To prepare the formulation add the triclabendazole and PEG 400. Warm to 70°C and stir to dissolve. Cool mix to less than 25°C and dilute to volume with PEG 400.

### Reference Example 41

| **Ingredients** | **Quantity** |
|---|---|
| Triclabendazole | 10g |
| Glycerol formal | 10g |
| PEG 400 q. s. to | 100 ml |

To prepare the formutation add the triclabendazole, glycerol formal and PEG 400. Warm to 70°C and stir to dissolve. Cool mix to less than 25°C and dilute to volume with PEG 400.

### Reference Example 42

| **Ingredients** | **Quantity** |
|---|---|
| Triclabendazole | 10g |
| Benzyl alcohol | 10g |
| PEG 400 q. s. to | 100 ml |

To prepare the formulation add the triclabendazole, benzyl alcohol and PEG 400. Warm to 70°C and stir to dissolve. Cool mix to less than 25°C and dilute to volume with PEG 400.

### Reference Example 43

| **Ingredients** | **Quantity** |
|---|---|
| Triclabendazole | 10g |
| Propylene glycol | 10g |
| PEG 400 q. s. to | 100 ml |

To prepare the formulation add the triclabendazole, propylene glycol and PEG 400. Warm to 70°C and stir to dissolve. Cool mix to less than 25°C and dilute to volume with PEG 400.

### Reference Example 44

| **Ingredients** | **Quantity** |
|---|---|
| Triclabendazole | 10g |
| Ethyl alcohol | 10g |
| PEG 400 q. s. to | 100 ml |

To prepare the formulation add the triclabendazole, ethyl alcohol and PEG 400. Warm to 70°C and stir to dissolve. Cool mix to less than 25°C and dilute to volume with PEG 400.

### Reference Example 45

| **Ingredients** | **Quantify** |
|---|---|
| Triclabendazole | 5 g |
| PEG 400 q. s. to | 100 ml |

To prepare the formulation add the triclabendazole and PEG 400. Warm to 70°C and stir to dissolve. Cool mix to less than 25°C and dilute to volume with PEG 400.

### Reference Example 46

| **Ingredients** | **Quantity** |
|---|---|
| Triclabendazole | 5g |
| Glycerol formal | 10g |
| PEG 400 q. s. to | 100 ml |

To prepare the formulation add the triclabendazole, glycerol formal and PEG 400. Warm to 70°C and stir to dissolve. Cool mix to less than 25°C and dilute to volume with PEG 400.

### Reference Example 47

| **Ingredients** | **Quantity** |
|---|---|
| Triclabendazole | 5g |
| Benzyl alcohol | 10g |
| PEG 400 q. s. to | 100 ml |

To prepare the formulation add the triclabendazole, benzyl alcohol and PEG 400. Warm to 70°C and stir to dissolve. Cool mix to less than 25°C and dilute to volume with PEG 400.

### Reference Example 48

| **Ingredients** | **Quantity** |
|---|---|
| Triclabendazole | 5g |
| Propylene glycol | 10g |
| PEG 400. q, s. to | 100 ml |

To prepare the formulation add the triclabendazole, propylene glycol and PEG 400. Warm to 70°C and stir to dissolve. Cool mix to less than 25°C and dilute to volume with PEG 400.

### Reference Example 49

| **Ingredients** | **Quantity** |
|---|---|
| Triclabendazole | 5g |
| Ethyl alcohol | 10g |
| PEG 400 q. s. to | 100 ml |

To prepare the formulation add the triclabendazole, ethyl alcohol and PEG 400. Warm to 70°C and stir to dissolve. Cool mix to less than 25°C and dilute to volume with PEG 400.

It will be appreciated that the above formulations are examples only and that other formulations are contemplated wherein the solvent system may include any combination of 2-pyrrolidone and/or liquid polyethylene glycol with additional solvents selected from the group comprising benzyl alcohol, propylene glycol, N-methyl pyrrolidone, glycerol formal, glycol ethers, butyl dioxitol, methyl alcohol, ethyl alcohol, isopropyl alcohol, 1-butanol and 1-hexanol.

Furthermore, it will be appreciated that the formulation may further include additional anthelmintic actives or other medicaments which are soluble in the solvent system. For example, the additional anthelmintic active or actives may be selected from the group comprising avermectins, milbemycins, tetramisole and levamisole. Abamectin has been found to be particularly suitable.

The formulation may also further include excipients such as dyes, preservatives, stabilises, buffers, thickeners, anti-foaming agents, spreading agents and the like.

### Stability Trials

Various formulations containing 2-pyrrolidone or PEG were prepared and stored at room temperature and refrigeration temperature for 30 days. The formulations showed no deposition at room temperature or under refrigeration.

### Efficacy Trials

To assess the field performance of the invention a study was undertaken to compare the bioavailability of a formulation of the present invention with that of a formulation of the commercially available triclabendazole pour-on formulation as described in PCT/NZ00/00053.

A high concentration 60% Triclabendazole pour-on formulation was made as described in Example 4 (with the addition of abamectin) and as shown below ('Formulation 1').

### Formulation 1

| **Ingredient** | **G/100ml** |
|---|---|
| Abamectin | 1 |
| Triclabendazole | 60 |
| N-methyl pyrrolidone | 10 |
| 2-pyrrolidone | To volume |

The commercially available lower concentration 30% pour-on formulation as described in PCT/NZ00/00053 has the following formulation ('Formulation 2').

### Formulation 2

| **Ingredient** | **G/100ml** |
|---|---|
| Abamectin | 0.5 |
| Triclabendazole | 30 |
| Benzyl alcohol | 5 |
| Diethyl glycol monobutyl ether | 50 |
| Polyethylene glycol | To volume |

The trial included ten animals divided into two groups of five animals. At day zero of the trial all animals were treated. Group 1 animals were treated with Formulation 2 and Group 2 animals were treated with Formulation 1. Both formulations were applied topically to the skin surface of the animal. To minimise the possibility of licking, all animals were treated on a shaved portion of the neck and isolated in individual paddocks for the duration of the trial.

Blood samples were taken at days 4, 6, 8 and 10. Comparative levels of the active triclabendazole sulphoxide (which is the metabolite that appears in the body as the triclabendazole breaks down) were measured in the animals and the results are shown in the following table 5 (measurements of triclabendazole sulphoxide are expressed in ng/ml).

The study measured comparative levels of the metabolite triclabendazole sulphoxide component in animals expressed in ng/ml. (Triclabendazole breaks down to the sulphoxide and the sulphone in the body).

Both formulations were applied topically and to minimise the possibility of licking all animals were treated on a shaved portion of the neck and isolated in individual paddocks for the duration of the trial. Blood samples were taken at Days 4, 6, 8, and 10

The results of the study clearly demonstrated that at least in terms of levels of triclabendazole sulphoxide the 60% formulation was capable of delivering a result comparable to the commercial 30% formulation.

**Table 2:**

| **Group 1 Commercially available Triclabendazole Pour-On** | | | | | | |
|---|---|---|---|---|---|---|
| Cattle ID No. | 2 | 127 | 250 | 1022 | 1038 | Average |
| Day 0 | <LOD | <LOD | <LOD | <LOD | <LOD | |
| Day 4 | 0.75 | 0.59 | 0.42 | 0.91 | 0.81 | 0.696 |
| Day 6 | 0.41 | 0.42 | 0.33 | 0.42 | 0.59 | 0.434 |
| Day 8 | 0.27 | 0.23 | 0.27 | 0.29 | 0.48 | 0.308 |
| Day 10 | 0.53 | 0.16 | 0.22 | 0.29 | 0.44 | 0.328 |
| AUC* (µg.d/mL) | 4.1 | 3.2 | 2.7 | 4.5 | 5 | 3.9 |

| **Group 2 60% Triclabendazole Pour-on Solution** | | | | | | |
|---|---|---|---|---|---|---|
| Cattle ID No. | 238 | 422 | 445 | 1041 | 9980 | |
| Day 0 | <LOD | <LOD | <LOD | <LOD | <LOD | |
| Day 4 | 0.32 | 1.84 | 0.46 | 0.2 | 0.38 | 0.64 |
| Day 6 | 0.49 | 0.42 | 0.54 | 0.31 | 0.43 | 0.438 |
| Day 8 | 0.36 | 0.37 | 0.4 | 0.2 | 0.57 | 0.38 |
| Day 10 | 0.31 | 0.68 | 0.39 | 0.55 | 0.38 | 0.462 |
| AUC* (µg.d/mL) | 3 | 7.8 | 3.6 | 2.2 | 3.5 | 4.02 |

| | | | | | | |
|---|---|---|---|---|---|---|
| * AUC — Area under concentration-time curve | | | | | | |

The results of the study clearly demonstrate that at least in terms of levels of triclabendazole sulphoxide the formulation of the present invention is capable of delivering a result comparable to Ancare New Zealand Limited's commercially available formulation as described in our PCT/NZ00/00053 which is known to provide effective treatment of parasitic infections, including fasciolisis.

### ADVANTAGES

The triclabendazole formulations of the present invention are advantageous as they allow the inclusion of triclabendazole in solution at significantly higher concentrations than previously known. This is advantageous as it allows the administration of an effective amount of triclabendazole in a low volume dose.

By way of example, pour-on formulations including triclabendazole in solution at about 40% w/v or greater will deliver an effective dose of triclabendazole to a 350 kg cow in about 25 ml of formulation. This smaller volume is particularly advantageous as it marries with the amount of liquid a pour-on gun can deliver. In addition, it increases the number of animals that may be treated out of the same pack thereby decreasing the need to change packs. The lower volume also further decreases the risk of the triclabendazole running off the back of the animal.

In field use these formulations would be highly desirable as they could provide the flexibility to deliver high concentrations of triclabendazole to an animal in a relatively small amount of solvent. Concentrated quantities of triclabendazole in solution could be delivered at a rate of as low as 1 ml per 20 kg of body weight, which equates to as little as 17.5 ml of formulation for a 350 kg cow.

### VARIATIONS

It is envisaged that the formulations of the invention may be modified by the inclusion of additional excipients.

In particular it may be desirable to include dyes in the formulation to allow easy identification of treated animals. Alternately other excipients such as preservatives, stabilisers, buffers, thickeners, anti-foaming agents, spreading agents and the like may be included to modify the formulation to specific animals.

Also, additional excipients may be included within the formulations of the present invention to tailor them to suit a specific administration method.

In this specification the words "includes", "including" and the like and "comprises", "comprising" and the like should be considered synonymous and be given a non-exhaustive meaning.

Finally it will be appreciated that various other alterations and modifications may be made to the foregoing without departing from the scope of the invention.

## Claims

1. A veterinary anthelmintic formulation containing a stable solution of triclabendazole, wherein the solution contains an effective amount of triclabendazole and a solvent system containing one or more solvents, wherein at least one of the solvents is selected from 2-pyrrolidone and liquid polyethylene glycol, and wherein the triclabendazole is present in the range of 40 - 60% w/v.

2. A veterinary anthelmintic formulation as claimed in claim 1, wherein the solvent system also includes one or more additional solvents selected from benzyl alcohol, propylene glycol, N-methyl pyrrolidone, glycerol formal, glycol ethers, butyl dioxitol, methyl alcohol, ethyl alcohol, isopropyl alcohol, 1-butanol and 1-hexanol.

3. A veterinary anthelmintic formulation according to claim 1 which is a pour-on formulation.

4. A veterinary anthelmintic formulation as claimed in claim 3, wherein the solvent system also includes one or more additional solvents selected from
benzyl alcohol, propylene glycol, N-methyl pyrrolidone, glycerol formal, glycol ethers, butyl dioxitol, methyl alcohol, ethyl alcohol, isopropyl alcohol, 1-butanol and 1-hexanol.

5. A veterinary anthelmintic formulation as claimed in claim 4, wherein the solvent system contains 2-pyrrolidone and at least one additional solvent selected from butyl dioxitol, N-methyl pyrrolidone, benzyl alcohol, glycerol formal, propylene glycol and ethyl alcohol.

6. A veterinary anthelmintic formulation as claimed in any one of claims 3 to 5, wherein the triclabendazole is present in a concentration such that an effective amount of triclabendazole may be delivered to an animal in a volume of substantially 25 ml or less.

7. A veterinary anthelmintic formulation as claimed in any previous claim, wherein the formulation includes at least one additional anthelmintic active or other medicament which is soluble in the solvent system.

8. A veterinary anthelmintic formulation as claimed in claim 7, wherein the additional anthelmintic active or actives are selected from avermectins, milbemycins, tetramisole and levamisole.

9. A veterinary anthelmintic formulation as claimed in claim 7 or claim 8, wherein the additional anthelmintic active is an avermectin, such as abamectin.

10. A veterinary anthelmintic formulation as claimed in any previous claim wherein the formulation further includes excipients such as dyes, preservatives, stabilisers, buffers, thickeners, anti-foaming agents or spreading agents.

11. A veterinary anthelmintic formulation as claimed in any previous claim wherein the formulation contains (a) about 60% w/v of triclabendazole, (b) about 1.0% w/v of abamectin, and a solvent system, wherein the solvent system includes N-methyl pyrrolidone in an amount of about 10% w/v and 2-pyrrolidone in an amount of about 29% w/v.

12. A veterinary anthelmintic formulation according to any one of claims 1 to 11 for use in treating parasites including fasciolisis in warm blooded animals.

13. A veterinary anthelmintic formulation according to claim 12 in the form of a pour-on formulation in a form suitable for administration in a volume of substantially 25 ml or less on to the skin surface of an animal for use according to claim 12.

14. A veterinary anthelmintic formulation according to claim 12 in the form of a pour-on formulation for use according to claim 12 wherein the formulation is to be administered in a volume capable of delivering at least lmg of triclabendazole per 10kg of body weight of the animal to be treated.

15. Use of a veterinary anthelmintic formulation according to any one of claims 1 to 11 in the preparation of a medicament for treating parasites including fasciolisis in warm blooded animals.

## Patentansprüche

1. Eine veterinärmedizinische anthelmintische Formulierung, die eine stabile Lösung von Trielabendazol enthält, wobei die Lösung eine wirksame Menge an Triclabendazol und ein ein oder mehrere Lösungsmittel enthaltendes Lösungsmittelsystem enthält, wobei mindestens eines der Lösungsmittel aus 2-Pyrrolidon und flüssigem Polyethylenglykol ausgewählt ist und wobei das Triclabendazol im Bereich von 40-60% Gew./Vol. vorliegt.

2. Eine veterinärmedizinische anthelmintische Formulierung wie in Anspruch 1 beansprucht, wobei das Lösungsmittelsystem auch ein oder mehrere zusätzliche Lösungsmittel, ausgewählt aus Benzylalkohol, Propylenglykol, N-Methylpyrrolidon, Glycerinformal, Glykolethern, Butyldioxitol, Methylalkohol, Ethylalkohol, Isopropylalkohol, 1-Butanol und 1-Hexanol, beinhaltet.

3. Eine veterinärmedizinische anthelmintische Formulierung nach Anspruch 1, bei welcher es sich um eine Pour-on-Formulierung handelt.

4. Eine veterinärmedizinische anthelmintische Formulierung wie in Anspruch 3 beansprucht, wobei das Lösungsmittelsystem auch ein oder mehrere zusätzliche Lösungsmittel, ausgewählt aus Benzylalkohol, Propylenglykol, N-Methylpyrrolidon, Glycerinformal, Glycolethern, Butyldioxitol, Methylalkohol, Ethylalkohol, Isopropylalkohol, 1-Butanol und 1-Hexanol, beinhaltet.

5. Eine veterinärmedizinische anthelmintische Formulierung wie in Anspruch 4 beansprucht, wobei das Lösungsmittelsystem 2-Pyrrolidon und mindestens ein zusätzliches Lösungsmittel, ausgewählt aus Butyldioxitol, N-Methylpyrrolidon, Benzylalkohol, Glycerinformal, Propylenglylcol und Ethylalkohol, enthält.

6. Eine veterinärmedizinische anthelinintische Formulierung wie in einem der Ansprüche 3 bis 5 beansprucht, wobei das Triclabendazol in einer derartigen Konzentration vorliegt, dass eine wirksame Menge an Triclabendazol einem Tier in einem Volumen von im Wesentlichen 25 ml oder weniger verabreicht werden kann.

7. Eine veterinärmedizinische anthelmintische Formulierung wie in einem vorhergehenden Anspruch beansprucht, wobei die Formulierung mindestens einen zusätzlichen anthelmintischen Wirkstoff oder ein anderes Medikament, das in dem Lösungsmittelsystem löslich ist, beinhaltet.

8. Eine veterinärmedizinische anthelmintische Formulierung wie in Anspruch 7 beansprucht, wobei der/die zusätzliche(n) anthelmintische(n) Wirkstoff(e) aus Avermectinen, Milbemycinen, Tetramisol und Levamisol ausgewählt ist/sind.

9. Eine veterinärmedizinische anthelmintische Formulierung wie in Anspruch 7 oder Anspruch 8 beansprucht, wobei der zusätzliche anthelmintische Wirkstoff ein Avermectin, wie z.B. Abamectin, ist.

10. Eine veterinärmedizinische anthelmintische Formulierung wie in einem vorhergehenden Anspruch beansprucht, wobei die Formulierung weiter Exzipienten, wie z.B. Farbstoffe, Konservierungsmittel, Stabilisatoren, Puffer, Verdiclcungsmittel, Entschäumer oder Verteilungsmittel, beinhaltet.

11. Eine veterinärmedizinische anthelmintische Formulierung wie in einem vorhergehenden Anspruch beansprucht, wobei die Formulierung (a) etwa 60% Gew./Vol. an Triclabendazol, (b) etwa 1,0% Gew./Vol. an Abamectin und ein Lösungsmittelsystem enthält, wobei das Lösungsmittelsystem N-Methylpyrrolidon in einer Menge von etwa 10% Gew./Vol. und 2-Pyrrolidon in einer Menge von etwa 29% Gew./Vol. beinhaltet.

12. Eine veterinärmedizinische anthelmintische Formulierung nach einem der Ansprüche 1 bis 11 zur Verwendung bei der Behandlung von Parasiten, einschließlich Fasciolisis, bei warmblütigen Tieren.

13. Eine veterinärmedizinische anthelmintische Formulierung nach Anspruch 12 in der Form einer Pour-on-Formulierung in einer zur Verabreichung in einem Volumen von im Wesentlichen 25 ml oder weniger auf die Hautoberfläche eines Tieres geeigneten Form zur Verwendung nach Anspruch 12.

14. Eine veterinärmedizinische anthelmintische Formulierung nach Anspruch 12 in der Form einer Pour-on-Formulierung zur Verwendung nach Anspruch 12, wobei die Formulierung in einem Volumen zu verabreichen ist, das in der Lage ist, mindestens 1 mg Triclabendazol pro 10 kg Körpergewicht des zu behandelnden Tieres zu verabreichen.

15. Verwendung einer veterinärmedizinischen anthelmintischen Formulierung nach einem der Ansprüche 1 bis 11 bei der Herstellung eines Medikaments zur Behandlung von Parasiten, einschließlich Fasciolisis, bei warmblütigen Tieren.

## Revendications

1. Formule vétérinaire anthelminthique contenant une solution stable de triclabendazole, dans laquelle la solution contient une quantité efficace de triclabendazole et un système de solvants contenant un ou plusieurs solvants, au moins un des solvants étant choisi parmi la 2-pyrrolidone et le polyéthylène glycol liquide et le triclabendazole étant présent dans la plage allant de 40 % en poids/volume à 60 % en poids/volume.

2. Formule vétérinaire anthelminthique selon la revendication 1, dans laquelle le système de solvant comprend également un ou plusieurs solvants supplémentaires choisis parmi l'alcool benzylique, le propylène glycol, la N-méthyl pyrrolidone, le glycérol formal, les éthers de glycol, le butyl dioxitol, l'alcool méthylique, l'alcool éthylique, l'alcool isopropylique, le 1-butanol et le 1-hexanol.

3. Formule vétérinaire anthelminthique selon la revendication 1, qui est une formule à verser.

4. Formule vétérinaire anthelminthique selon la revendication 3, dans laquelle le système de solvants comprend également un ou plusieurs solvants supplémentaires choisis parmi l'alcool benzylique, le propylène glycol, la N-méthyl pyrrolidone, le glycérol formal, les éthers de glycol, le butyl dioxitol, l'alcool méthylique, l'alcool éthylique, l'alcool isopropylique, le 1-butanol et le 1-hexanol.

5. Formule vétérinaire anthelminthique selon la revendication 4, dans laquelle le système de solvants contient de la 2-pyrrolidone et au moins un solvant supplémentaire choisi parmi le butyl dioxitol, la N-méthyl pyrrolidone, l'alcool benzylique, le glycérol formal, le propylène glycol et l'alcool éthylique.

6. Formule vétérinaire anthelminthique selon l'une quelconque des revendications 3 à 5, dans laquelle le triclabendazole est présent à une concentration telle qu'une quantité efficace de triclabendazole puisse être administrée à un animal dans un volume inférieur ou sensiblement égale à 25 ml.

7. Formule vétérinaire anthelminthique selon l'une quelconque des revendications précédentes, dans laquelle la formule comprend au moins une substance anthelminthique active supplémentaire ou un autre médicament qui est soluble dans le système de solvants.

8. Formule vétérinaire anthelminthique selon la revendication 7, dans laquelle la substance anthelminthique active supplémentaire ou les substances actives sont choisies parmi les avermectines, les milbémycines, le tétramisole et le lévamisole.

9. Formule vétérinaire anthelminthique selon la revendication 7 ou la revendication 8, dans laquelle la substance anthelminthique active supplémentaire est une avermectine, telle que l'abamectine.

10. Formule vétérinaire anthelminthique selon l'une quelconque des revendications précédentes, dans laquelle la formule comprend en outre des excipients, tels que des colorants, des conservateurs, des stabilisants, des tampons, des épaississants, des agents anti-mousses ou des agents d'enduction.

11. Formule vétérinaire anthelminthique selon l'une quelconque des revendications précédentes, dans laquelle la formule contient (a) environ 60 % en poids/volume de triclabendazole, (b) environ 1,0 % en poids/volume d'abamectine et un système de solvants, le système de solvants comprenant de la N-méthyl pyrrolidone en une quantité d'environ 10 % en poids/volume et de la 2-pyrrolidone en une quantité d'environ 29 % en poids/volume.

12. Formule vétérinaire anthelminthique selon l'une quelconque des revendications 1 à 11, utilisable pour traiter des parasites, comme la fascioliase, chez les animaux à sang chaud.

13. Formule vétérinaire anthelminthique selon la revendication 12, sous la forme d'une formule à verser sous une forme appropriée pour être administrée dans un volume inférieur ou sensiblement égal à 25 ml sur la surface cutanée d'un animal, pour un usage selon la revendication 12.

14. Formule vétérinaire anthelminthique selon la revendication 12, sous la forme d'une formule à verser pour un usage selon la revendication 12, dans laquelle la formule doit être administrée dans un volume capable de délivrer au moins 1 mg de triclabendazole pour 10 kg de poids corporel de l'animal à traiter.

15. Utilisation d'une formule vétérinaire anthelminthique selon l'une quelconque des revendications 1 à 11, dans la préparation d'un médicament destiné à traiter des parasites, comme la fascioliase, chez les animaux à sang chaud.
